# EUROPEAN PATENT SPECIFICATION

(11) **EP 0 465 345 B1**
(45) Date of publication and mention of the grant of the patent: **08.05.1996**
(21) Application number: 91401809.8
(22) Date of filing: 02.07.1991
(51) Int. Cl.: A61B 5/022

(54) **Blood pressure measuring apparatus**
Blutdruckmessgerät
Appareil pour mesurer la tension artérielle

(30) Priority: 03.07.1990 JP 176158/90; 03.07.1990 JP 176159/90
(43) Date of publication of application: 08.01.1992
(73) Proprietor: UEDA ELECTRONIC WORKS LIMITED, Bunkyo-ku, Tokyo (JP)
(72) Inventor: Sengoku, Masafumi, c/o UEDA ELEC. WORKS LTD., Bunkyo-ku, Tokyo (JP)
(74) Representative: Signore, Robert

(56) References cited:
- EP-A- 0 078 090
- EP-A- 0 423 553
- US-A- 4 779 626
- MEDICAL PROGRESS THROUGH TECHNOLOGY vol. 12, no. 1-2, 1987, pages 123-143, Dordrecht, NL; KEN-ICHI YAMAKOSHI et al.: " Noninvasive measurement of arterial blood pressure and elastic properties using photoelectric pletzysmography technique"

## Description

### Background of the invention

### (Field of the invention)

The present invention relates to a blood pressure measuring apparatus, and more particularly to a blood pressure measuring apparatus by means of which highly accurate blood pressure values can be obtained.

### (Prior Art)

Primarily, we speak of blood pressure as being in the heart or aorta, but it is physically difficult to measure blood pressure in these areas ; therefore, the measurement of blood pressure has been carried out in the areas of the head, upper arm, abdomen, thigh, or finger. MEDICAL PROGRESS TECHNOLOGY, vol. 12, N°1-2, 1987, pages 123-143, Dordrecht, the Netherlands ; Kenichi Yamakoshi et al. : "Noninvasive measurement of arterial blood pressure and elastic properties using photoelectric plethysmography technique" and US-A-4,779,626 disclose devices for blood pressure measurement by means of correcting measured blood pressure in accordance with a hydrostatic pressure difference between a selected point at which blood pressure is measured and the heart. EP-A-0 078 090 relates to a method for controlling the cuff pressure in the indirect, non-invasive and continous measurement of the blood pressure.

However, in the case in which there is external light or noise resulting from alternating current induction, there is a problem in that reliable blood pressure measurement may not be obtained.

### Summary of the invention

The present invention solves the above-stated problems : it is an object of the present invention to provide a blood pressure measuring apparatus which allows reliable blood pressure values to be obtained in accordance with a true signal. The blood pressure measuring apparatus of the present invention comprises a cuff for pressing a predetermined part of a living body ; a light-emitting element ; a light-receiving element provided in such a way that the light-receiving element faces the light-emitting element across the predetermined part of the living body ; a blood pressure measurement means for obtaining a blood pressure value of the living body based on an amount of light received by the light-receiving element when light emitted from the ligth-emitting element is transmitted through the predetermined part of the living body inserted in the cuff and is received by the light-receiving element ; a lighting control means for intermittently driving the light-emitting element ; a memory means having (i) a first memory part for storing a first value of the amount of light received by the light-receiving element when the light-emitting element is lighted and (ii) a second memory part for storing a second value of the amount of light received by the light-receiving element when the light-emitting element is not lighted ; a subtracting means for subtracting the second value from the first value and outputting a subtracted result ; and a blood pressure calculating means for calculating the blood pressure based on the subtracted result outputted by the subtracting means.

In accordance with the blood pressure measuring apparatus of the present invention, the light-emitting element is intermittently driven. The value of the amount of light received by the light-receiving element when the light-emitting element is not lighted is subtracted from the value of the amount of light received by the light-receiving element when the light-emitting element is lighted. Then, the blood pressure of the living body is calculated based on the subtracted result.

Accordingly, the above-mentioned external light or noise can be excluded and only the true signal related to blood pressure is obtained.

### Brief description of the drawings

Figure 1 is an angled view from the outside of a blood pressure measuring apparatus according to an embodiment of the present invention.

Figure 2 is a block figure showing an outline of the composition of a blood pressure measuring apparatus according to the same preferred embodiment.

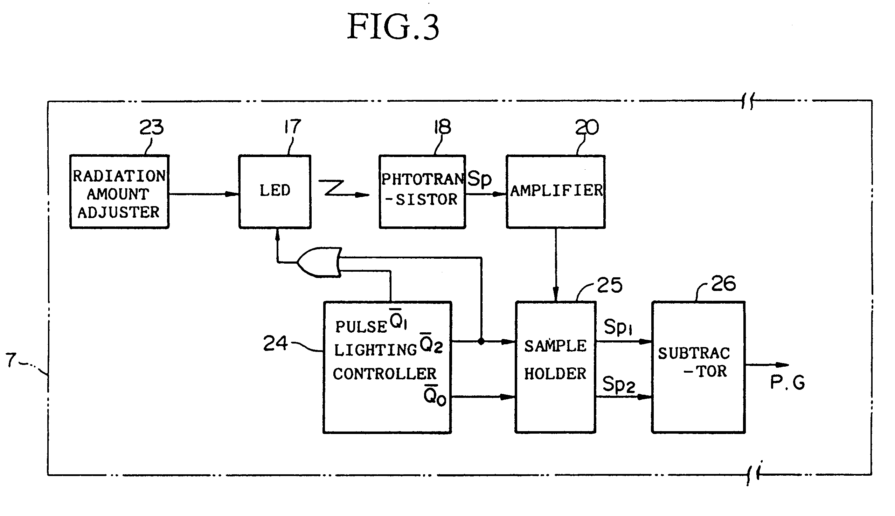

Figure 3 is an angled view showing the outside of a chest unit comprising a blood pressure measuring apparatus according to the same preferred embodiment.

Figure 4 is a block diagram showing a blood pressure measurement circuit of the same preferred embodiment.

Figure 5 is a time chart for the purpose of explaining the operation of the blood pressure measurement circuit of the same preferred embodiment.

### Detailed Description of the Preferred Embodiments

Hereinafter, the preferred embodiment will be explained with reference to the diagrams.

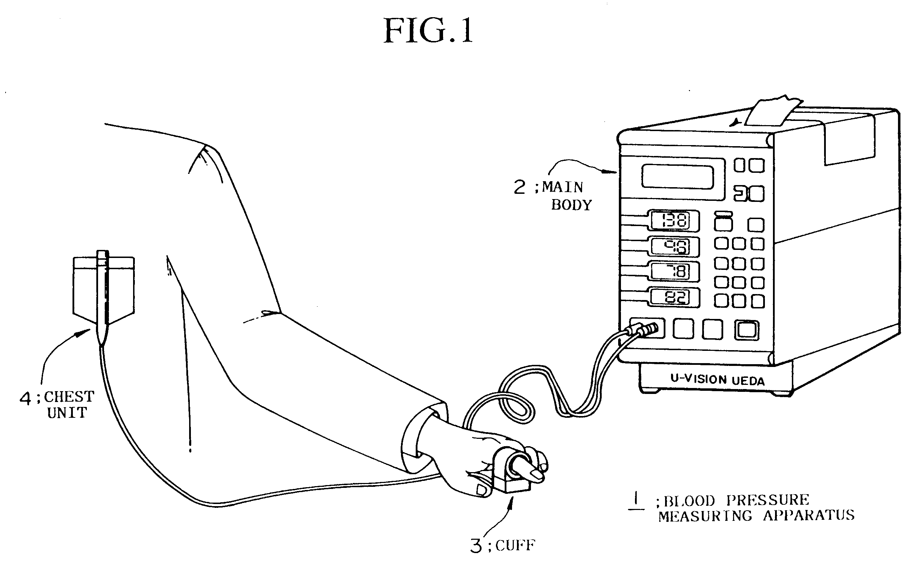

Figure 1 is an angled view showing the outside of a blood pressure measuring apparatus 1 according to a preferred embodiment of the present invention. As shown in the diagram, the blood pressure measuring apparatus 1 is comprised of apparatus main body 2, cuff 3, and chest unit 4.

Blood pressure measuring apparatus 1 uses the venous pulse waveform method, conducts blood pressure measurement and pulse measurement, and calculates maximum blood pressure, average blood pressure, minimum blood pressure, and pulse rate using the results of these measurements. Furthermore, the results of these calculations are displayed on a display screen and outputted to a printer.

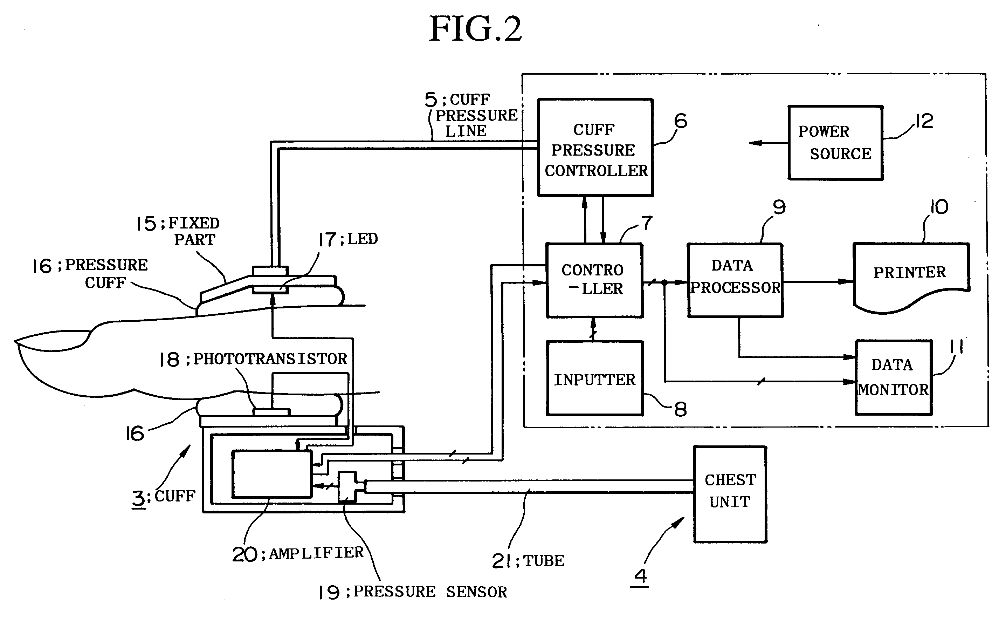

Figure 2 is a block diagram showing the construction of blood pressure measuring apparatus 1. In the diagram, apparatus main body 2 is provided with cuff pressure controller 6, controller 7, inputter 8, data processor 9, printer 10, data monitor 11, and power source 12.

Cuff pressure controller 6 sends air to cuff 3 through cuff pressure line 5. Controller 7 controls all parts of the apparatus, reads the signals outputted from cuff 3 converts these signals to digital, and outputs them. This controller 7 is provided with a CPU (Central Processing Unit)(not shown), a ROM (Read Only Memory), in which the program for controlling this CPU is stored, a RAM (Random Access Memory), an interface, and a blood pressure measuring circuit. A detailed description of the blood pressure measuring circuit will be given hereinbelow.

Inputter 8 inputs each type of data (weight, height, sex, etc.). Data processor 9 finds maximum blood pressure, average blood pressure, minimum blood pressure, and pulse rate, based on the data outputted by controller 7. Printer 10 prints the data outputted by data processor 9. Data monitor 11 displays the data outputted by controller 7 and data processor 9. Power source 12 supplies power to all parts of the blood pressure measuring apparatus.

Cuff 3 is provided with a cylindrical-form fixed part 15 which surrounds a finger, a pressure cuff 16 attached to the inner side of fixed part 15, an LED 17 attached to the upper part of the inner side of fixed part 15, a phototransistor 18 attached to the bottom part of the inner side of fixed part 15 and facing this LED 17, and an amplifier 20, which amplifies the the output of phototransistor 18.

The sensitivity of pressure sensor 19 can be adjusted by means of the adjustment of the amplification rate of amplifier 20. LED 17 and phototransistor 18 are used in the detection of the blood pressure and pulse rate based on the pulse waveform method mentioned above. The lighting of this LED 17 is carried out by controller 7. Pressure sensor 19 detects the pressure impressed by chest unit 4 (explained in detail hereinbelow).

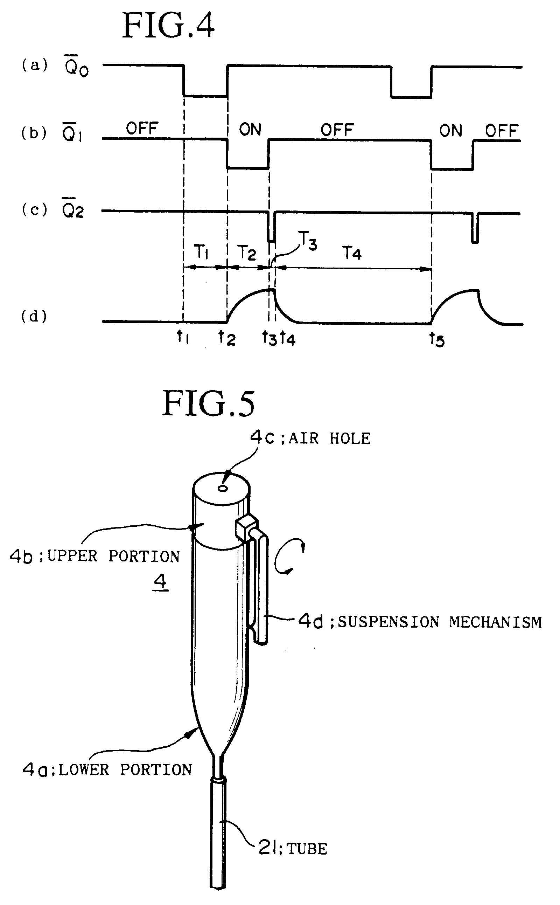

Diagram 3 is a block diagram showing the blood pressure measuring circuit of controller 7. In the diagram, radiation amount adjuster 23 adjusts the amount of emitted light of LED 17. Pulse lighting controller 24 controls the timing of the emission of light by LED 17. This pulse lighting controller 24 outputs signals according to the timing shown in Figures 4 (a)-(c). During period T₁ from time t₁ to time t₂, terminal Q-bar₀ is set to "L", and next, during period T₂ from time t₂ to time t₃, terminal Q-bar₁ is set to "L". Then, during period T₃ from time t₃ to time t₄, terminal Q-bar₂ is set to "L",
and during periods T₃ and T₄from time t₃ to time t₅, terminal Q-bar₁ is set to "H". After this, the same process is repeated. In this case, the on/off frequency of LED 17 caused by pulse lighting controller 24 is greater than the frequency in the body (rough figure in direct current - several hundred Hz), and is set as an integral multiple of the commercial power frequency (50 Hz or 60 Hz). In the case in which both 50 Hz and 60 Hz commercial power frequencies are used, the on/off frequency is set as an integral multiple of the product of these two frequencies (here, 300 Hz, therefore, 300, 600, 900, 1200,...Hz). Furthermore, pulse lighting controller 24 enables the adjustment of the duty ratio of the on and off states of LED 17; by means of this adjustment, the division of the lighted periods and non-lighted periods can be freely set.

Phototransistor 18 detects the light which is emitted by LED17 and passes through the body, and outputs a signal Sp with a level which corresponds to the amount of light received. It is acceptable to use a photodiode or a CDS instead of phototransistor 18. Amplifier 20 amplifies the signal Sp outputted by photodiode 18 to a fixed level, as described above, and outputs it. Sample holder 25 samples the signal outputted by amplifier 20 based on the signal outputted by pulse lighting controller 24 and holds it. Subtractor 26 carries out the subtraction of the signal Sp₁ of the non-lighted period and the signal Sp₂ of the lighted period which are held in sample holder 25 and outputs the results.

Diagram 5 is an angled view showing the above-mentioned chest unit 4. As shown in the diagram, chest unit 4 has a tubular shape and its lower portion 4a is tapered. Furthermore, the upper portion 4b of chest unit 4 can be freely detached so as to permit the entry of a liquid (water or oil) into the same unit 4. In addition, an air hole 4c is opened in this upper portion 4c as shown in the diagram. A valve, not shown in the diagram, is attached to this air hole 4c for the purpose of preventing fluid leakage. One end of a tube 21 is attached to the opened end of the lower portion 4a of chest unit 4. As shown in Diagram 2, the other end of this tube 21 is attached to pressure sensor 19. A suspension mechanism 4d is attached to the upper portion 4b of this chest unit 4 so as to allow the suspension of the same chest unit 4 from a shirt pocket or the like. This suspension mechanism 4d is freely rotatable in a vertical direction with respect to the main body of this same unit 4 and thus keeps the main body of the same unit 4 always facing in a vertical direction. The chest unit 4 with this construction is suspended from a shirt pocket or the like at the place where the heart is located, and thus pressure corresponding to the height from the heart to the finger is exerted on the detection surface of pressure sensor 19.

Next, the operation of the blood pressure measuring apparatus according to the above construction will be explained.

After the chest unit 4 has been suspended from a shirt pocket, the upper portion 4b of the same unit 4 is removed, and a liquid poured in until the surface of the liquid reaches the level of the heart. Next, the sensitivity of the pressure sensor 19 is set to a value which corresponds to the ratio of the specific gravities of the fluid and blood. Next, a finger is inserted into cuff 3, and a measurement initiation switch (not shown in the diagram) is moved to the on position. By means of this, air is supplied to the pressurizing cuff 16, and the finger is placed under pressure. In this period, the measurement of blood pressure is carried out. Hereinbelow, the blood pressure measurement operation is explained with reference to Diagram 4.

After the measurement initiation switch is set to the on position, from times t₁ to t₂ of period T₁, terminal Q-bar₀ of pulse lighting controller 24 is set to "L", and a signal, corresponding to the signal Sp₁ of phototransistor 18 while LED 17 is off, is sampled and held by sample holder 25. Then, from times t₂ to t₃ of period T₂, terminal Q-bar₁ of pulse lighting controller 24 is set to "L", and LED 17 is lit. The waveform received by phototransistor 18 when LED 17 is lit is as shown in Figure 4 (d); it rises gradually from time t₂, reaches a peak at time t₄, and falls rapidly after this. The signal Sp₂ supplied when this LED 17 is lit is sampled and held by sample holder 25. Next, the signal Sp₂ and the signal Sp₁ which are held herein are supplied to subtractor 26 and the subtraction (Sp₂-Sp₁) is carried out. By means of this subtraction, the signal caused by external light, including the signal Sp₂ received when LED 17 is lit, or the signal caused by alternating current induction, is subtracted from the signal caused by external light including the signal Sp₁ received when LED 17 is not lit, or the signal caused by alternating current induction, and the desired signal P.G is obtained. This signal P.G corresponds to the blood pressure value in the finger. This signal P.G is converted to digital data, along with the signal outputted by pressure sensor 19, and supplied to data processor 9.

Data processor 9 subtracts the hydrostatic pressure difference (the hydrostatic pressure difference between the finger and the heart obtained by means of pressure sensor 19) from the blood pressure value in the finger. By means of this, the measurement differences occurring as a result of the difference in height between the finger and the heart are corrected. In this case, as the finger is in a lower position than the heart, the blood pressure value in the finger is higher than that in the heart. Accordingly, by subtracting the hydrostatic pressure difference between the finger and the heart from the blood pressure value in the finger, the blood pressure value in the heart can be obtained.

In the above embodiment, LED 17 is pulse-lit, so that as greater electrical current can be passed through LED 17 than in the case in which it is continuously lit. Accordingly, this embodiment has an advantage in that it is possible to increase the sensitivity.

Furthermore, in the above embodiment, the frequency at which LED 17 turns on and off is set as an integral multiple of the commercial power frequency (50 Hz or 60 Hz), or as an integral multiple of the smallest product of these two frequencies, and the signal Sp₂ received when LED 17 is not lit is subtracted from the signal Sp₁ received when LED 17 is lit, so that there is an advantage in that external light or noise resulting from alternating current induction is excluded and only the true signal is obtained.

In addition, in the above-mentioned embodiment, the sampling of the signal SP₁ received when LED 17 is not lit occurs directly before the lighting of the same LED 17, so that there is an advantage in that the signal Sp₁ is obtained under conditions in which there is no residual light from LED 17.

### (Other Preferred Embodiments)

1). In the above-mentioned embodiment, the finger was used as the place at which blood pressure was measured, but if cuffs of other forms corresponding to other places were made, blood pressure values corresponding to any place could be obtained. In the case in which a place which is lower than the heart is selected as the measurement place, as with the above-mentioned finger, the hydrostatic pressure difference between the heart and the finger is subtracted from the blood pressure value obtained as a result of the measurement. On the other hand, in the case in which a place which is higher than the heart is selected as the measurement place, the hydrostatic pressure difference between the heart and this place is added to the blood pressure value obtained as a result of the measurement.
2). Furthermore, in the above-mentioned embodiment, LED 17 was used, but it is also possible to use a lamp or the like.
3). In addition, in the above-mentioned embodiment, phototransistor 18 was used, but it is also possible to use a photodiode or a CDS or the like.
4). Furthermore, when a fluid having the same specific gravity as blood is used as the solution for the detection of hydrostatic pressure differences, and a differential-type pressure sensor is used as the pressure sensor, even if the adding and subtracting processes in the above embodiment are not carried out, it is possible to measure blood pressure while always correcting for hydrostatic pressure differences.
5). In addition, by making the frequency at which the LED 17 turns on and off sufficiently higher than that of the changes occurring inside the body (for example, several hundred Hz or more), it is possible to raise the exclusion rate of alternating current induction (hum). Furthermore, as the frequency component of external light can be thought of as direct current or in tens of Hz, by setting the frequency at which LED 17 turns on and off to hundreds of Hz or more, it is possible to eliminate the external light component.

## Claims

1. A blood pressure measuring apparatus comprising :
a cuff (3) for pressing a predetermined part of a living body ;
a light-emitting element (17) ;
a light-receiving element (18) provided in such a way that the light-receiving element faces the light-emitting element across the predetermined part of the living body ;
a blood pressure measurement means for obtaining a blood pressure value of the living body based on an amount of light received by the light-receiving element when light emitted from the light-emitting element is transmitted through the predetermined part of the living body inserted in the cuff and is received by the light-receiving element ;
a memory means (25) ; and
a blood pressure calculating means (9) ;
said blood pressure measuring apparatus being characterized by :
further comprising a lighting control means (24) for intermittently driving the light-emitting element (17) ;
the memory means (25) having (i) a first memory part for storing a first value of the amount of light received by the light-receiving element (18) when the light-emitting element (17) is lighted and (ii) a second memory part for storing a second value of the amount of light received by the light-receiving element when the light-emitting element is not lighted ;
further comprising a subtracting means (26) for subtracting said second value from said first value and outputting a substracted result ; and
the blood pressure calculating means (9) being adapted for calculating said blood pressure based on the subtracted result outputted by the subtracting means.

2. A blood pressure measuring apparatus in accordance with claim 1, characterized by further comprising :
a hydrostatic-pressure difference detecting means for detecting a hydrostatic-pressure difference between the predetermined part of the living body and a heart part of the living body ; and
a correcting means for adding the hydrostatic-pressure difference outputted from hydrostatic-pressure difference detecting means to the blood pressure value calculated by the blood pressure calculating means (9), or substracting the hydrostatic-pressure difference from the blood pressure, on the basis of a positional relationship between the predetermined part and the heat part of the living body.

3. A blood pressure measuring apparatus in accordance with claim 1, characterized in that a lighting/not-lighting repeating frequency of the light-emitting element (17) is greater than a frequency of a physiological change within the living body, and is an integral multiple of a commercial power frequency.

4. A blood pressure measuring apparatus in accordance with claim 1, characterized in that the integral multiple of the commercial frequency is an integral multiple of the least common multiple of different commercial power frequencies.

## Patentansprüche

1. Blutdruckmeßgerät, enthaltend:
eine Manschette (3) zum Zusammendrücken eines vorbestimmten Teils eines lebenden Körpers;
ein lichtemittierendes Element (17);
ein lichtempfangendes Element (18), das auf eine solche Weise vorgesehen ist, daß das lichtempfangene Element dem lichtemittierenden Element quer über den vorbestimmten Teil des lebenden Körpers gegenüberliegt;
eine Blutdruckmeßeinrichtung zur Gewinnung eines Blutdruckwertes des lebenden Körpers auf der Basis eines von dem lichtempfangenden Element empfangenen Lichtbetrages, wenn von dem lichtemittierenden Element emittiertes Licht durch den vorbestimmten Teil des lebenden Körpers übertragen wird, der in die Manschette eingeführt ist, und von dem lichtempfangenden Element empfangen wird;
eine Speichereinrichtung (25); und
eine Blutdruck-Berechnungseinrichtung (9); wobei die Blutdruck-Berechnungseinrichtung (9) dadurch gekennzeichnet ist, daß:
sie weiterhin eine Lichtsteuereinrichtung (24) zum intermittierenden Ansteuern des lichtemittierenden Elementes (17) aufweist;
die Speichereinrichtung (25) (i) einen ersten Speicherteil zum Speichern eines ersten Wertes des von dem lichtempfangenden Element (18) empfangenen Lichtbetrages, wenn das lichtemittierende Element (17) leuchten gelassen wird, und (ii) einen zweiten Speicherteil zum Speichern eines zweiten Wertes des von dem lichtempfangenden Element empfangenen Lichtbetrages aufweist, wenn das lichtemittierende Element nicht leuchten gelassen wird;
sie weiterhin eine Subtrahiereinrichtung (26) zum Subtrahieren des zweiten Wertes von dem ersten Wert und zum Ausgeben eines Subtraktionsergebnisses aufweist; und
die Blutdruck-Berechnungseinrichtung (9) dafür eingerichtet ist, den Blutdruck auf der Basis des von der Subtrahiereinrichtung ausgegebenen Subtraktionsergebnisses zu berechnen.

2. Blutdruckmeßgerät gemäß Anspruch 1, dadurch gekennzeichnet, daß es weiterhin enthält:
eine Detektoreinrichtung für eine hydrostatische Druckdifferenz, zur Erfassung einer hydrostatischen Druckdifferenz zwischen dem vorbestimmten Teil des lebenden Körpers und einem Herzteil des lebenden Körpers; und
eine Korrektureinrichtung zum Addieren der von der Detektoreinrichtung für eine hydrostatische Druckdifferenz ausgegebenen hydrostatischen Druckdifferenz zu dem mittels der Blutdruck-Berechnungseinrichtung (9) berechneten Blutdruckwert oder zum Subtrahieren der hydrostatischen Druckdifferenz von dem Blutdruck auf der Basis einer Positionsbeziehung zwischen dem vorbestimmten Teil und dem Herzteil des lebenden Körpers.

3. Blutdruckmeßgerät gemäß Anspruch 1, dadurch gekennzeichnet, daß eine Leucht/Nichtleucht-Wiederholfrequenz des lichtemittierenden Elementes (17) größer als eine Frequenz einer physiologischen Änderung innerhalb des lebenden Körpers ist und ein ganzzahliges Vielfaches einer Netzstromfrequenz ist.

4. Blutdruckmeßgerät gemäß Anspruch 1, dadurch gekennzeichnet, daß das ganzzahlige Vielfache der Netzfrequenz ein ganzzahliges Vielfaches des kleinsten gemeinsamen Vielfachen von unterschiedlichen Netzstromfrequenzen ist.

## Revendications

1. Appareil de mesure de la pression sanguine, comprenant :
un fourreau (3) pour presser une partie prédéterminée d'un corps vivant ;
un élément émetteur de lumière (17) ;
un élément récepteur de lumière (18), placé de telle manière que l'élément récepteur de lumière soit situé en face de l'élément émetteur de lumière, de part et d'autre de la partie prédéterminée du corps vivant ;
un moyen de mesure de la pression sanguine, servant à obtenir une valeur de la pression sanguine du corps vivant, sur la base d'une quantité de lumière reçue par l'élément récepteur de lumière, lorsque la lumière émise par l'élément émetteur de lumière est transmise à travers la partie prédéterminée du corps vivant, introduite dans le fourreau, et qu'elle est reçue par l'élément récepteur de lumière ;
un moyen formant mémoire (25) ; et
un moyen de calcul de la pression sanguine (9) ; ledit appareil de mesure de la pression sanguine étant caractérisé par :
le fait qu'il comprend, en outre, un moyen de commande d'allumage (24) pour attaquer, de manière intermittente, l'élément émetteur de lumière (17) ;
que le moyen formant mémoire (25) comporte (i) une première partie de mémoire pour conserver une première valeur de la quantité de lumière, reçu par l'élément récepteur de lumière (18) lorsque l'élément émetteur de lumière (17) est allumé, et (ii) une seconde partie de mémoire pour conserver une seconde valeur de la quantité de lumière, reçue par l'élément récepteur de lumière lorsque l'élément émetteur de lumière n'est pas allumé ;
le fait qu'il comprend en outre un moyen soustracteur (26) pour soustraire ladite seconde valeur de ladite première valeur et pour sortir un résultat de la soustraction ; et
que le moyen de calcul de la pression sanguine (9) est propre à calculer ladite pression sanguine, sur la base du résultat de la soustraction, sorti par le moyen soustracteur.

2. Appareil de mesure de la pression sanguine selon la revendication 1, caractérisé en ce qu'il comprend en outre :
un moyen détecteur de différence de pression hydrostatique, destiné à détecter une différence de pression hydrostatique entre la partie prédéterminée du corps vivant et la partie coeur du corps vivant ; et
un moyen correcteur servant à ajouter la différence de pression hydrostatique, fournie par le moyen détecteur de différence de pression hydrostatique, à la valeur de la pression sanguine, calculée par le moyen de calcul de la pression sanguine (9), ou à soustraire de la pression sanguine, la différence de pression hydrostatique sur la base d'une relation d'emplacement entre la partie prédéterminée et la partie coeur du corps vivant.

3. Appareil de mesure de la pression sanguine selon la revendication 1, caractérisé en ce qu'une fréquence de répétition d'allumage/de non-allumage de l'élément émetteur de lumière (17) est plus grande que la fréquence d'une modification physiologique à l'intérieur du corps vivant, et en ce qu'elle est un multiple entier d'une fréquence industrielle.

4. Appareil de mesure de la pression sanguine selon la revendication 1, caractérisé en ce que le multiple entier de la fréquence industrielle est un multiple entier du plus petit commun multiple de différentes fréquences industrielles.
